# EUROPEAN PATENT APPLICATION

(11) **EP 1 018 560 A1**
(43) Date of publication of application: **12.07.2000**
(21) Application number: 98929813.8
(22) Date of filing: 02.07.1998
(51) Int. Cl.: C12N 15/85, C12N 5/10, A61K 48/00

(54) **VECTORS FOR TREATING CANCER**

(30) Priority: 20.08.1997 JP 22365197
(71) Applicant: Dnavec Research Inc., Ibaraki 305-0856 (JP)
(72) Inventor: YOKOI, Haruhiko, Tsukuba-shi, Ibaraki 305-0856 (JP); TAKEDA, Katsuo, DECEASED (JP); HASEGAWA, Mamoru, Tsukuba-shi, Ibaraki 305-0856 (JP)
(74) Representative: VOSSIUS & PARTNER
(86) International application number: JP9802993
(87) International publication number: WO9909191

(57) **Abstract**

A technique for gene expression specific to cells free from a specific transcription factor, which comprises constructing a recombinase expression unit wherein a recombinase gene is located in the downstream of a promoter depending on the specific transcription factor and another unit wherein two target sequences of a desired gene to be expressed and the recombinase are located in the downstream of another promoter; and infecting cells with these units. According to this technique, in cells carrying the specific transcription factor, the recombinase is expressed and thus recombination occurs between the recognition sequences, whereby the target gene is not expressed but deleted. In cells free from the specific transcription factor, on the other hand, the recombinase is not expressed and thus no recombination occurs between the recognition sequences, which allows the expression of the target gene.

## Description

### Technical Field

The present invention relates to a technique for gene expression specific to cells free from a specific transcription factor, and its application to treating cancer; it thus belongs to the field of gene therapy.

### Background Art

Cancer is a disorder in which normal mechanisms controlling cell proliferation go awry due to mutation of somatic cells, viral infection, etc. This results in the uncontrolled growth of cells and their further invasion of adjacent tissues and occupation thereof, eventually leading to the death of individuals. Cell growth is controlled by the balance between activities of the growth accelerator gene and the growth suppressor gene. Changes in genes alter mechanisms of controlling proliferation in most cancer cells. Specifically, such changes are either abnormally elevated activity of the accelerator gene (also referred to as the protooncogene, e.g. ras, myc, and abl) due to the mutation or inactivation of the suppressor gene (also referred to as the tumor suppressor gene, e.g. Rb, p53, and APC) due to mutation, viral gene action, etc. Among these cancer-associated genes, the tumor suppressor gene p53 is mutated in about half of cancer cells hitherto examined. The correlation between the functional loss of p53 and oncogenesis has thus drawn considerable attention (Levine et al., 1997, Cell, **88**, 323-331).

With the recent progress in the development of vehicles (vectors) for DNA molecules, gene therapy has become possible. Gene therapy's application to cancer therapy has therefore been studied as its most significant objective. Gene therapy strategies are roughly classified into those using: 1) a method for enhancing the immunity against cancer cells by transferring the cytokine gene, 2) a method enabling more potent chemotherapy by transferring a protective gene such as MDR into bone marrow cells to reduce their high sensitivity to antitumor agents, and 3) a method for transferring a gene leading cancer cells to suicide (Davis et al., 1996, Curr. Opin. Oncol., **8**, 499-508). Of these strategies, selectivity, that is, a high cytotoxic effect on tumor cells but practically no cytotoxicity toward normal cells, is desired, especially in the third strategy. For that purpose, a method for expressing a (suicide) gene specific to tumor cells has been developed.

A method utilizing a promoter of a gene highly expressed in cancer cells compared with normal cells is a typical gene expression system specific to cancer cells. For example, it has been demonstrated that the promoter for the AFP (α-fetoprotein) gene that is highly expressed in many hepatoma (Tamaoki, et al., 1995, Tanpakushitu Kakusan Koso (Protein, Nucleic Acid and Enzyme), **40**, No. 17, 2613-2617), the promoter for the tyrosine kinase gene that is highly expressed in many of melanoma (Vile et al., 1993, Cancer Res., **53**, 3860-64), the promoter for the erbB2 gene that is highly expressed in many breast and pancreas cancers (Harris et al., 1994, Gene Ther., **1**, 170-175), and the promoter for the prostate specific antigen (PSA) gene that is highly expressed in many prostate cancers (Pang et al., 1995, Hum. Gene Ther., **6**, 1417-26) direct the gene expression specific to their corresponding cancer types. However, these promoters apply only to a very limited number of types of cancers, and such promoters have not been identified for all cancers. Furthermore, absolute amounts of gene expression directed by these promoters are not always high.

Attempts have also been made to apply p53, the functional abnormality of which has been recognized in many of cancers, to a gene expression system specific to cancer. The recently disclosed system of Balmain et al. (Balmain et al., WO97/12970) is one example. This system features the use of two units. One unit contains a promoter that is highly expressed in cancer cells (or cells without the p53 function) (e.g. a promoter of the hsp70 gene) to direct the expression of a desired gene. The second unit expresses a gene (such as a sequence-specific transcription suppressor, ribozyme, or anti-sense gene) to down-regulate the expression of the desired gene under the control of a p53-dependent promoter. When these two units are introduced into normal cells, the activity of the promoter directing the expression of a desired gene is weak, and a suppressor (or antisense, ribozyme, etc.) gene dependent on p53 is expressed. This also suppresses the expression of the desired gene. In contrast, when these two units are introduced into cancer cells, the activity of the promoter directing the expression of the desired gene is strong. Furthermore, the expression of the p53-dependent transcription suppressor (or antisense, ribozyme, etc.) is low, so the expression of the desired gene is not suppressed. Balmain et al. have expected, therefore, that these double effects can express genes specific to cancer cells. However, examples disclosed in this publication indicate a low specificity. One possible cause of the low specificity is the means for suppressing the post-transcription steps (transcription suppressor, ribozyme, and antisense) for down-regulating the expression of a desired gene. Such means can produce some degree of effects when the promoter connected to the target gene does not give rise to its high expression. However, the effects are reduced as the promoter potency increases. Therefore, these means cannot be expected to provide the high expression intensity and specificity desired for practical gene therapy.

A gene expression system specific to cancer cells to which the properties of the Cre-loxP recombination system of *Escherichia coli* are combined has also been designed (Sato et al., 1997, the 3rd Annual Meeting of the JAPAN Society of Gene Therapy, Abstract p49). Cre is a site-specific DNA recombinase derived from *Escherichia coli* P1 phage, and loxP is the target sequence thereof. Since Cre recognizes the loxP sequence to perform recombination of DNA molecules between two loxPs, it is possible to turn the gene expression switch ON/OFF in both *Escherichia coli* and mammalian cells using this property (Kilby et al., 1993, Trends Genet., **9**, 413-421). A Switch ON system is employed in the expression system of Sato et al. to express the target gene by Cre. In particular, there are two units. One unit expresses the Cre gene under the control of an AFP promoter specific to hepatoma cells; the other unit has a sequence of loxP-transcription termination signal-loxP inserted between a gene to be expressed and its promoter. When these two units are transferred into hepatoma cells in which the Cre expression is high, sequences between two loxPs are frequently recombined, detaching the transcription termination signal and enhancing the expression of the desired gene. In contrast, when these two units are introduced into cells other than hepatoma cells in which the Cre expression is low, sequences between loxPs are scarcely recombined, so that the expression of the desired gene is also low. These results are thought to indicate a formation of an expression system in which the expression intensity (of a desired gene) is enhanced 50- to 100-fold with the AFP promoter specificity being almost completely maintained. This system overcomes the problem in the expression intensity, but the problem in the application range has not been solved. Furthermore, in such a switch ON system, the expression of the desired gene becomes enhanced with the lapse of time due to the slight Cre expression by AFP promoter even in cells other than hepatoma cells (about 1/100 that in hepatoma cells), posing a problem in specificity. As a result, taking the suicide gene as the desired gene, for example, there is concern about possibly elevating the cytotoxicity toward normal cells.

### Disclosure of the Invention

An objective of the present invention is to provide a technique for expressing a desired gene specific to cells free from a specific transcription factor. Using the technique, it is a further objective of this invention to provide a technique for gene expression highly specific to cancer cells and scarcely harmful to normal cells.

The present inventors studied how to solve the above problems and found a technique for expressing genes specific to cells free from a specific transcription factor. This technique comprises constructing two recombinase expression units. In one, a recombinase gene is located downstream of a promoter depending on the specific transcription factor. In the other, a gene to be expressed and two target sequences of the recombinase are located downstream of another promoter. These units are then used to infect cells. This technique expresses recombinase in cells carrying the specific transcription factor. Recombination thus occurs between the recognition sequences, whereby the target gene is not expressed but deleted. In contrast, the recombinase is not expressed in cells free from the specific transcription factor, thus no recombination occurs between the recognition sequences, allowing the expression of the target gene.

Furthermore, the present inventors, as a result of study to apply this gene expression technique to cancer therapy, succeeded in selectively exterminating cancer cells. We did this by using a promoter depending on the tumor suppressor as the gene depending on a specific transcription factor and a gene specifically toxic to cancer cells, such as a suicide gene, as the gene to be expressed. They were thus able to express a gene specifically toxic to cancer cells (cells free from a tumor suppressor).

This invention therefore relates to a technique for gene expression specific to cells free from a specific transcription factor and its application to cancer therapy, more specifically to:
1. a vector comprising (1) a promoter functioning in the presence of a specific transcription factor and a gene encoding a recombinase present under the control of the promoter, and (2) an expressible desired gene and two target sequences of the recombinase present near the gene, wherein the desired gene and the two target sequences are so located that the desired gene will not be expressed when recombination occurs between the two target sequences,
2. the vector according to 1, in which (1) a promoter functioning in the presence of a specific transcription factor and a gene encoding a recombinase present under the control of the promoter, and (2) an expressible desired gene and two target sequences of the recombinase present near the gene are present in the same molecule,
3. the vector according to 1, in which (1) a promoter functioning in the presence of a specific transcription factor and a gene encoding a recombinase present under the control of the promoter, and (2) an expressible desired gene and two target sequences of the recombinase present near the gene are present in the different molecules,
4. the vector according to 1, in which the desired gene is interposed between two target sequences of the recombinase,
5. the vector according to 4, in which a promoter controlling the desired gene is interposed between two target sequences of the recombinase,
6. the vector according to 1, in which the specific transcription factor is a translation product of a tumor suppressor gene,
7. the vector according to 6, in which the tumor suppressor gene is the p53 gene,
8. the vector according to 1, in which the recombinase is Cre, and the target sequence of recombinase is loxP,
9. the vector according to 1, in which the desired gene is a gene encoding a suicide enzyme,
10. the vector according to 1, in which the specific transcription factor is a tumor suppressor gene product, and the desired gene is a gene encoding a suicide enzyme,
11. the vector according to 10, in which the tumor suppressor gene is the p53 gene,
12. a host cell transformed with the vector according to any one of 1 to 11, and
13. a method for selectively exterminating cells free from a specific transcription factor, the method comprising introducing the vector according to any one of 1 to 11 into host cells *in vitro*.

In this invention, "transcription factor" refers to a protein having the activity to stimulate the expression of a specific gene, but does not include a protein that has lost the transcription activity; "vector" includes both a single vector and a two different vectors set; and "suicide gene" refers to a gene encoding a protein or RNA leading cells to death, including a gene encoding a protein or RNA leading cells to death by itself (e.g., a gene for a toxin such as diphtheria toxin and apoptosis-inducing gene such as bax) and a gene for an enzyme converting a compound with a low cytotoxicity to one with a high toxicity (e.g., a gene for thymidine kinase derived from herpes simplex virus and a gene for cytosine deaminase derived from *Escherichia coli*).

The vector according to this invention contains (1) a promoter functioning in the presence of a specific transcription factor and a gene encoding a recombinase present under the control of the promoter (referred to as the "first unit" hereafter), and (2) an expressible desired gene and two target sequences of the recombinase present near the gene (referred to as the "second unit" hereafter).

If a transcription factor that activates a promoter on the vector is present in cells to be transfected with the vector, the first unit expresses the recombinase gene. The expressed recombinase subsequently causes recombination between the two recognition sequences located near an desired gene in the second unit, so that the desired gene is irreversibly rendered inexpressible. In contrast, if a transcription factor that activates the promoter is not present in cells to be transfected with the vector, the recombinase gene is not expressed in the first unit, so the second unit expresses the desired gene.

Therefore, the vector of this invention enables suppressing the expression of a desired gene in cells carrying a specific transcription factor and the desired gene in cells free from the specific transcription factor. In this case, the first unit and second unit may be present either in a single molecule of a vector or in different molecules of vectors.

There is no particular limitation on the "specific transcription factor" acting on the promoter in the first unit of the vector according to this invention provided it is not present in desired cells in which an desired gene in the second unit is to be expressed, but is present in cells in which the expression of the desired gene is to be suppressed. For example, the p53 protein known for its mutation in many cancer cells, WT1 mutated in the Wilms' tumor and acute leukemia (Haber et al., 1990, Cell, **61**, 1257-69; and Underwood et al., 1996, Blood, **87**, 2171-79), Smad4 mutated in many pancreas tumors (Liu et al., 1997, Genes & Dev., **11**, 3157-67), and IRF-1 deleted in leukemia (Willman et al., 1993, Science, **259**, 968-71) can be used as transcription factors with the tumor suppressing function. If p53 is used as the transcription factor, promoters of the *WAF1* gene (El-Diery et al., 1993, Cell, **75**, 817-825), *bax* gene (Miyashita et al., 1993, Cell, **80**, 293-299), etc., which are under the control of the p53, or promoters having an artificially synthesized p53-binding consensus sequence (El-Diery et al., 1992, Nature Genet., **1**, 45-49) can be used. If WT1 is used as the transcription factor, for example, the promoter of the *Syndecan 1* gene that is expressed WT1-dependently (Cook et al., 1996, Oncogene, **13**, 1789-99) can be used. If Smad4 is used as the transcription factor, for example, the promoter of the plasminogen activator-inhibitor 1 gene (Dennler et al., 1998, Embo J., **17**, 3091-3100) can be used. If IRF-1 is used as the transcription factor, for example, the promoter of the double-stranded RNA-dependent protein kinase gene (Beretta et al., 1996, Oncogene, **12**, 1593-96) can be used. The "gene encoding recombinase" under the control of the promoter includes genes for the recombinase Cre of *Escherichia coli* P1 phage, recombinase FLP of *Saccharomyces cerevisiae*, and recombinase R of *Zygosaccharomyces rouxii* (Kilby et al., 1993, Trends Genet., **9**, 413-421). Preferably, a gene for Cre with an excellent recombination efficiency at 37°C is used (Buchholz et al., 1996, Nucleic Acids Res., **24**, 4256-4262).

The expressible desired gene in the second unit of the vector according to this invention is usually located downstream of a promoter. Any promoter (even one containing a so-called enhancer portion) can be used in the second unit as long as it functions in animal cells. Promoters yielding strong expression in a wide variety of cells are preferable. These include, for example, those of the *E1a* gene (Zabner et al., 1996, Gene Ther., **3**, 458-465) and the early gene of cytomegalovirus (Doll et al., 1996, Gene Ther., **3**, 437-447), and the CAG promoter in which the enhancer of the early gene of cytomegalovirus is linked to the promoter of the chicken actin gene (Niwa et al., 1991, Gene, **108**, 193-200). Depending on the purpose, a promoter producing a strong tissue-specific expression can be used. When a tumor suppressor binds to the promoter in the first unit, suicide genes such as the diphtheria toxin A-chain gene (Maxwell et al., 1986, Cancer Res., **46**, 4660-4664), the thymidine kinase gene derived from herpes simplex virus (Field et al., 1983, Proc. Natl. Acad. Sci. USA, **80**, 4139-4143), and the cytosine deaminase gene derived from *Escherichia coli* (Mullen et al., 1992, Proc. Natl. Acad. Sci. USA, **89**, 33-37) can be used in order to kill cancer cells.

The "target sequences of recombinase," can be, for example, the loxP sequence if Cre is used as the recombinase, the FRT sequence if FLP is used as the recombinase, and the R-target sequence if R (Kilby et al., 1993, Trends Genet., **9**, 413-421) is used as the recombinase. Target sequences of recombinase are located such that the above-described desired gene cannot be expressed when recombination occurs between the two target sequences. For example, two target sequences may be located so as to sandwich the desired gene or a promoter to control the expression of the desired gene.

The adenovirus vector (Miyake et al., 1996, Proc. Natl. Acad. Sci. USA, **93**, 1320-1324 and Kanegae et al., 1996, Acta Paediatr. Jpn., **38**. 182-188), retrovirus vector (Wakimoto et al., 1995, Tanpakushitsu Kakusan Koso (Protein, Nucleic Acid and Enzyme), **40**, 2508-2513), adeno-associated virus vector (Tamayori et al., 1995, Tanpakushitsu Kakusan Koso (Protein, Nucleic Acid and Enzyme), **40**, 2532-2538), plasmid vector (Zerrouqi et al., 1996, Cancer Gene Ther., **3**, 385-392), etc. can be inserted with these two units for gene therapy. These vectors can be efficiently produced by previously established methods.

Host cells to be introduced with vectors used for cancer therapy are, for example, cells at the tumor focus (and, consequently, normal cells near them). More specifically, if the vector according to this invention is used for cancer therapy, the p53-dependent promoter is used as the promoter, and the thymidine kinase gene derived from herpes simplex virus, a suicide gene, is employed as the gene in the second unit. This vector is delivered directly or intravascularly, for example, to tumor foci lacking p53. After a predetermined time, ganciclovir, the substrate for the suicide gene product, is administered around the tumor focus or systemically. In cancer cells expressing the suicide gene, ganciclovir becomes toxic leading to cell death. In normal cells where the suicide gene is not expressed, however, the cells do not die.

This vector can also be used for selectively exterminating p53-deficient cancer cells *ex vivo* in the case of transplantation of bone marrow cells derived from a cancer patient for the potentiation of cancer chemotherapy (Eckert et al., 1996, Blood, **88**, 3407-3415) or other purposes.

The activity of p53 has been known to be under various controls, responding to alterations of intracellular and extracellular environments. For example, UV irradiation, antitumor agents, hypoxia, high fever, and cell aging, etc. enhance the p53 activity. Intracellular factors such as mdm2, proteasome, and calpain are also known to reduce the p53 activity, while ref1, cyclin E, CBP, etc. elevate the p53 activity (Levine et al., 1997, Cell, **88**, 323-331; Jayaraman et al., 1997, Genes & Dev., **11**, 558-570; Gu et al., 1997, Nature, **387**, 819-827; and Selivanova et al., 1997, Nature Med., **3**, 632-638).

Therefore, using such information on controlling p53 activity, for example, by administering antitumor agents, it is possible to enhance the p53 activity in p53-carrying normal cells, to further elevate the expression-suppressing effects due to the Cre-loxP reaction, and to increase the difference in the expression between normal and cancer cells.

### Brief Description of the Drawings

Figure 1 shows the process for constructing pRLSVb-p53RE8.
Figure 2 shows the process for constructing the p53-dependent Cre expression unit.
Figure 3 shows the process for constructing the reporter unit.
Figure 4 is a graph showing effects of AxYp53RECre on the ganciclovir (GCV)-sensitivity in p53-deficient and p53-expressing cells infected with AxCAYLTK.

### Best Mode for Carrying Out the Invention

In the following, this invention will be described more specifically with reference to examples. However, this invention is not to be construed to be limited by these examples.

### Example 1 Construction of vectors

### 1) Construction of p53-dependent expression unit

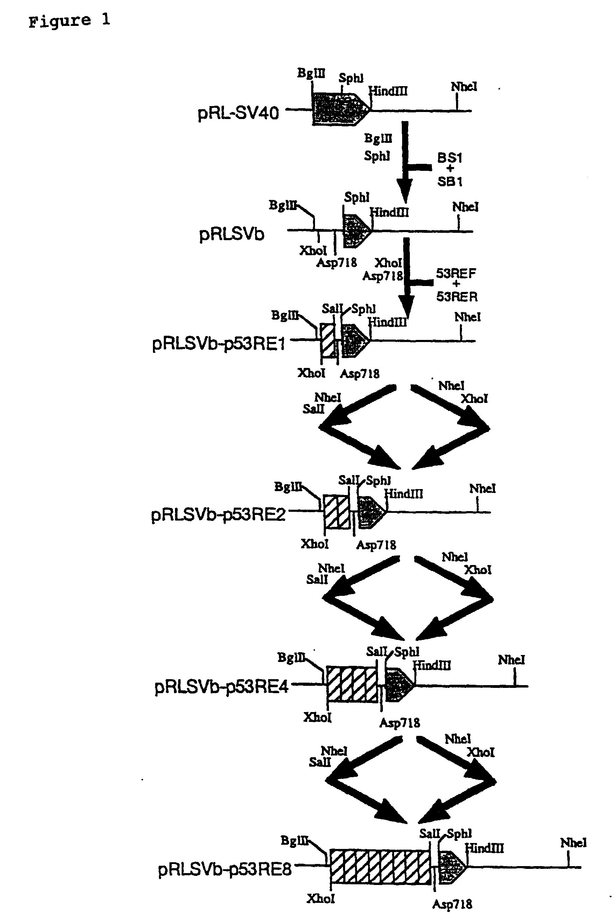

### 1-1) Preparation of p53-binding region (Fig. 1)

Synthetic oligonucleotide BS1 (GATCTCTCGAGCCCGGGGGTACCGCATG; SEQ ID NO: 1) and synthetic oligonucleotide SB1 (CGGTACCCCCGGGCTCGAGA; SEQ ID NO: 2), containing recognition sequences of restriction enzymes BglII, XhoI, SmaI, Asp718, and SphI, were treated with T4 polynucleotide kinase to phosphorylate the 5'-termini. The plasmid pRL-SV40 containing the SV40 early promoter (Promega, USA) was cleaved with restriction enzymes BglII and SphI to isolate a 3.5 kb fragment containing the basal promoter portion of the SV40 early promoter but not the enhancer portion. BS1 and SB1 were annealed, and ligated to the 3.5 kb fragment. Nucleotide sequences of the sequence derived from the synthetic oligonucleotides were determined to select a clone bearing no sequence error. This plasmid was designated as "pRLSVb."

Synthetic oligonucleotides 53REF (TCGAGGGACTTGCCTGGACTTGCCTGT CGACG; SEQ ID NO: 3) and 53RER (GTACCGTCGACAGGCAAGTCCAGGCAAGTCCC; SEQ ID NO: 4), containing the p53 recognition sequence, were treated with T4 polynucleotide kinase to phosphorylate the 5'-termini, and then both of them were annealed. The annealed product has termini that are the same sequences as cohesive end sequences of XhoI and Asp718, and also has the SalI recognition sequence adjacent to the Asp718 cohesive end sequence. This annealed product was cloned between the restriction enzymes XhoI-Asp718 sites of pRLSVb. Nucleotide sequences of inserts were determined, and a clone bearing no sequence error was selected. This plasmid was designated as "pRLSVb-p53RE1."

pRLSVb-p53RE1 was cleaved with SalI and NheI (having one recognition site in the pRLSVb plasmid) to isolate a 3.0 kb fragment containing the p53-recognition sequence, the ampicillin resistance gene, and the plasmid replication origin. pRLSVb-p53RE1 was also cleaved with XhoI and NheI to isolate a 0.5 kb fragment containing the p53-recognition sequence. Both fragments were ligated to prepare a plasmid having two copies of the p53-recognition sequence located between the XhoI-Asp718 sites of pRLSVb. This plasmid was designated as "pRLSVb-p53RE2."

Next, pRLSVb-p53RE2 was cleaved with SalI and NheI to isolate a 3.0 kb fragment containing two copies of the p53-recognition sequence, the ampicillin resistance gene, and the plasmid replication origin. pRLSVb-p53RE2 was also cleaved with XhoI and NheI to isolate a 0.5 kb fragment containing two copies of the p53-recognition sequence. Both fragments were ligated to prepare a plasmid containing four copies of the p53-recognition sequence located between the XhoI-Asp718 sites of pRLSVb. This plasmid was designated as "pRLSVb-p53RE4."

Furthermore, pRLSVb-p53RE4 was cleaved with SalI and NheI to isolate a 3.1 kb fragment containing four copies of the p53-recognition sequence, the ampicillin resistance gene, and the plasmid replication origin. pRLSVb-p53RE4 was also cleaved with XhoI and NheI to isolate a 0.6 kb fragment containing four copies of the p53-recognition sequence, and both fragments were ligated. As a result, a plasmid having eight copies of the p53-recognition sequence located upstream (between the XhoI-Asp718 sites) of the basal promoter derived from the SV40 early promoter of the pRLSVb was prepared. This plasmid was designated as "pRLSVb-p53RE8."

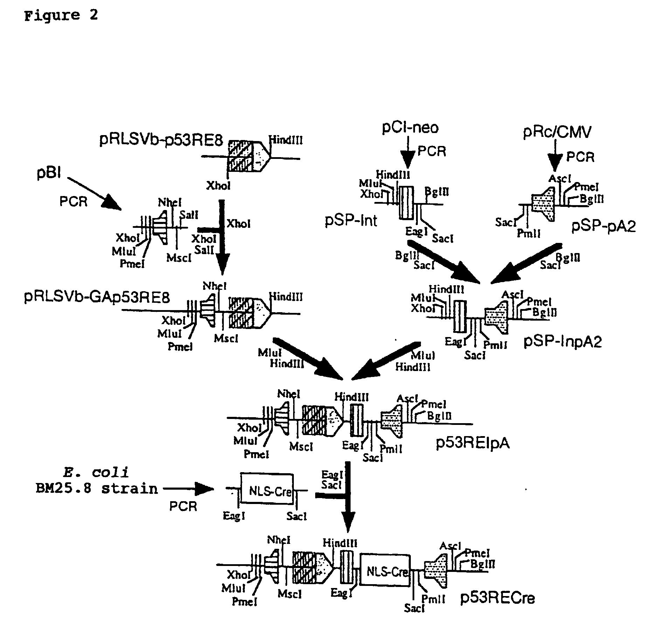

### 1-2) Cloning of transcription termination region of rabbit β -globin gene (Fig. 2)

PCR was performed on the plasmid pBI (Clontech, USA) as the template to amplify and isolate a region (0.25 kb) containing the transcription termination site of the rabbit β-globin gene in pBI using the synthetic oligonucleotide RGF (TCCCTCGAGACGCGTGTTTAAACCAATGCCCTGGCTCACAAATACC; SEQ ID NO: 5) containing the recognition sequences of restriction enzymes XhoI, MluI, and PmeI, and synthetic oligonucleotide RGR (TCCGTCGACTGGCCAGCTAGCGGCATATGTTGCCAAACTCTAAAC; SEQ ID NO: 6) containing the recognition sequences of restriction enzymes SalI, MscI, and NheI as the primers. This product was cleaved with restriction enzymes SalI and XhoI, and cloned into the XhoI site of the plasmid pRLSVb-p53RE8. Nucleotide sequences of inserts were determined, and a clone having the direction of the transcription termination signal coinciding with that of the promoter and bearing no mutation was selected. This plasmid was designated as "pRLSVb-GAp53RE8."

### 1-3) Cloning of introns (Fig. 2)

PCR was performed on the plasmid pCI-neo (Promega) as the template to amplify and isolate a region (0.35 kb) including introns of the human β-globin gene and human immunoglobulin gene in the pCI-neo using the synthetic oligonucleotide InF (TCCCTCGAGACGCGTAAGCTTTATTGCGGTAGTTTATCA; SEQ ID NO: 7) containing the recognition sequences of restriction enzymes XhoI, MluI, and HindIII, and synthetic oligonucleotide InR (TCCGAGCTCCGGCCGAGTACTCTAGCCTTAAGAGCTGTA; SEQ ID NO: 8) containing the recognition sequences of restriction enzymes SacI and EagI as the primers. This product was cleaved with the restriction enzymes SacI and XhoI, and cloned between the SacI-XhoI sites of the plasmid pSP72 (2.4 kb; Promega). Nucleotide sequences of inserts were determined, and a clone bearing no mutation was selected. This plasmid was designated as "pSP-Int."

### 1-4) Cloning of transcription termination region of the bovine growth hormone gene (Fig. 2)

The present inventors used the synthetic oligonucleotide PA2F (TCCGAGCTCCACGTGCTGTGCCTTCTAGTTGCCAGC; SEQ ID NO: 9) containing the recognition sequences of restriction enzymes SacI and PmlI, and synthetic oligonucleotide PA2R (TCCAGATCTGTTTAAACGGCGCGCCAAAGCCCCAAAAACAGGAAGA; SEQ ID NO: 10) containing the recognition sequences of restriction enzymes BglII, PmeI, and AscI as the primers to perform PCR on plasmid pRc/CMV (Invitrogen, USA) as the template to amplify and isolate a region (0.8 kb) including the transcription termination site of the bovine growth hormone gene in pRc/CMV. This product was cleaved with the restriction enzymes SacI and BglII, and cloned between the SacI-BglII sites of plasmid pSP72. Nucleotide sequences of inserts were determined, and a clone bearing no mutation was selected. This plasmid was designated as "pSP-pA2."

### 1-5) Coupling of each unit (Fig. 2)

pSP-pA2 was cleaved with the restriction enzymes SacI and BglII to isolate a 0.8 kb fragment containing the transcription termination site of the bovine growth hormone gene. pSP-Int was similarly cleaved with the restriction enzymes SacI and BglII to isolate a 2.8 kb fragment containing the intron, replicon, and ampicillin-resistance gene in the plasmid. Both fragments were ligated to prepare a plasmid containing the intron and transcription termination site of the bovine growth hormone gene. This plasmid was designated as "pSP-InpA2."

pRLSVb-GAp53RE8 was cleaved with restriction enzymes MluI and HindIII to isolate a 0.8 kb fragment containing the transcription termination site of the rabbit β-globin gene, p53-binding site, and SV40 basal promoter. This fragment was cloned between the MluI-HindIII sites (located on the 5'-side of the intron) of pSP-InpA2. This plasmid was designated as "p53REIpA." p53REIpA contains a unit having the transcription termination site of the rabbit β-globin gene (1) p53 binding site-SV40 basal promoter-intron (2), and transcription termination site of bovine growth hormone gene (3), in this order. This plasmid has recognition sites of three restriction enzymes (EagI, SacI, and PmlI) between (2) and (3).

### 1-6) Insertion of genes (Fig. 2)

### 1-6a) Construction and insertion of nuclear localization signal (NLS)-Cre gene

The present inventors used the synthetic oligonucleotide CreF 
(TGAAAGCTTCGGCCGCCACCATGCCCAAGAAGAAGCGCAAGGTGTCCAATTTACTGACCGTAC
ACCAA;  SEQ  ID  NO:  11)
 containing the nucleotide sequence corresponding to the nuclear localization signal peptide (PKKKRKV) derived from SV40 and recognition sequences of restriction enzymes HindIII and EagI, and synthetic oligonucleotide CreR (TCCGAGCTCCTAATCGCCATCTTCCAGCAGGCG; SEQ ID NO: 12) containing the recognition sequence of restriction enzyme SacI as the primers to perform PCR on DNA prepared from the *Escherichia coli* BM25.8 strain (Amersham, England) as the template. The BM25.8 strain is the lysogenic strain infected with P1 phage and has the Cre gene. PCR-amplified products were isolated, cleaved with restriction enzymes EagI and SacI, and cloned between the EagI-SacI sites of p53REIpA. Nucleotide sequences of inserts were determined, and a clone bearing no mutation was selected. This plasmid was designated as "p53RECre."

### 1-6b) Insertion of LacZ gene

pCMVbeta (Clontech) was cleaved with the restriction enzymes NotI and PstI to isolate a 3.5 kb DNA fragment. The fragment was cloned into the EagI site of p53REIpA. After the restriction enzyme cleavage pattern of inserts was examined, the clone with the fragment inserted in the desired direction was selected. This plasmid was designated as "p53RELZ."

### 1-7) Preparation of recombinant adenovirus vector containing units

Recombinant adenovirus vector was prepared basically according to the method of Saito et al. (Miyake et al., 1996, Proc. Natl. Acad. Sci. USA, **93**, 1320-24; Kanegae et al., Biomanual Series 4 - Idenshi Dounyu to Hatsugen-Kaisekihou (Method of Gene Transfer, Expression, and Analysis), 1994, 43-58, Yohdosha). Specific examples will be described below.

The 42 kb cosmid pAdex1cw containing a 31 kb AdDNA corresponding approximately to the full-length adenovirus (Ad) type 5 genome deprived of the E1 and E3 regions (Kanegae et al., 1994, Saibo Kogaku (Cell Technology), **13**, No. 8, 757-763) was cleaved with SwaI and desalted by gel filtration. p53RECre or p53RELZ was then cleaved with PmeI to isolate the cleaved product (2.9 or 5.3 kb) containing a p53-dependent Cre or LacZ gene expression unit. After both products were subjected to ligation reaction with T4 ligase, the enzyme was heat-inactivated, and the reaction product was desalted by gel filtration then cleaved with SwaI. After the enzyme was inactivated by treatment with phenol, cleaved products were desalted and digested again with SwaI. A portion of the products was subjected to *in vitro* packaging using a Gigapack XL kit (Stratagene, USA), and the ligated product was transfected into *Escherichia coli*. Cosmids were prepared from some of the ampicillin-resistant transformant strains thus obtained. Their structures were then examined by digestion with restriction enzymes to isolate the recombinant cosmids with the p53-dependent expression unit inserted in the desired direction (reverse direction of transcription of E1A and E1B). These cosmids were designated as "pAdp53RECre" or "pAxYp53RELZ."

The DNA of adenovirus type 5 deprived of E1 and E3 regions (Ad5 d1X strain) terminal protein complex (DNA-TPC) was prepared by the method of Saito et al. (Kanegae et al., 1994, Biomanual series 4 - Idenshi Dounyu to Hatsugen-Kaisekihou (Methods of Gene Transfer, Expression, and Analysis), p43-58, Yohdosha). This complex was digested with the restriction enzyme EcoT22I then desalted by gel filtration.

pAxYp53RECre (or pAxYp53RELZ) and EcoT22I-digested Ad5 dlXDNA-TPC were mixed and transfected into the 293 cells using a Cellfect kit (Pharmacia, Sweden). On the following day, transfected 293 cells were suspended, and that suspension and 10-fold to 100-fold dilutions thereof (diluted with a solution used for suspending the 293 cells) were plated into 96-well plates. About two weeks later, the culture medium containing dead cells was withdrawn from wells where proliferation of the recombinant adenovirus generated by recombination within the 293 cells was observed. The medium was freeze-thawed several times to release adenoviral particles from cells. The 293 cells placed in 24-well plates were infected with the centrifuged supernatant thereof (the primary viral solution). After three days, the culture medium containing dead cells was withdrawn. A portion thereof was freeze-thawed in a similar manner to that for the primary viral solution, and centrifuged to obtain the supernatant (secondary viral solution). The remaining culture medium was centrifuged to collect cells. DNA was prepared from cells thus obtained, and the structure of recombinant adenovirus DNA was examined by cleavage with restriction enzymes to select clones in which the desired structure was confirmed. These clones were designated as "AxYp53RECre" and "AxYp53RELZ." A larger amount of 293 cells was then infected with the secondary viral solutions thereof. The culture medium thereof was freeze-thawed similarly as before, and centrifuged to obtain the tertiary viral solution. The tertiary viral solution was assayed for its titer by the method of Saito et al. (Kanegae et al., 1994, Biomanual Series 4 - Idenshi Dounyu to Hatsugen-Kaisekihou (Method of Gene Transfer, Expression, and Analysis), p43-58, Yohdosha).

### 2) Preparation of recombinant adenovirus vector for p53 expression

### 2-1) Cloning of p53 cDNA

Single-stranded cDNAs were synthesized using poly A(+) RNA derived from the human testis (Clontech) as the templates and oligo-dT as the primer with a Superscript-Preamplification System (Gibco-BRL, USA). Using that mixture of cDNAs as the template, the current inventors performed PCR using the oligonucleotides P53F (AGCTTCTAGACAGCCAGACTGCCTTCCGGGTCACTGC; SEQ ID NO: 13) and P53R (GTTCTAGACCCCATGTAATAAAAGGTG; SEQ ID NO: 14), both containing the recognition sequence of restriction enzyme XbaI, as the primers. Amplified products were cleaved with restriction enzyme XbaI to isolate a DNA fragment (1.7 kb) containing the open reading frame of p53, and the fragment was cloned into the XbaI site of the pBluescriptII SK(-) plasmid (Stratagene). Nucleotide sequences of inserts were determined, and a clone bearing no mutation on the amino acid level was selected. This plasmid was designated as "pBS-p53."

### 2-2) Preparation of recombinant adenovirus vector

pBS-p53 was cleaved with XbaI to isolate a fragment containing p53cDNA, and its terminus was blunted with a DNA blunting kit (Takara Shuzo, Japan). The 44 kb cosmid pAdex1CA containing a 31 kb AdDNA corresponding to approximately full-length Ad type 5 genome deprived of the E1 and E3 regions and the CAG promoter-transcription termination region (Kanegae et al., 1994, Saibo Kogaku (Cell Technology), **13**, No. 8, 757-763) was cleaved with SwaI and desalted by gel filtration.

Using both the p53cDNA and cosmid fragments, a recombinant cosmid was prepared in a manner similar to that of 1-7). This cosmid was designated as "pAxCAYp53." Using this cosmid, a recombinant adenovirus having the CAG promoter-p53 gene-transcription termination region was prepared by the method described in 1-7). This recombinant adenovirus was designated as "AxCAYp53."

### 3) Construction of adenovirus vector for reporter

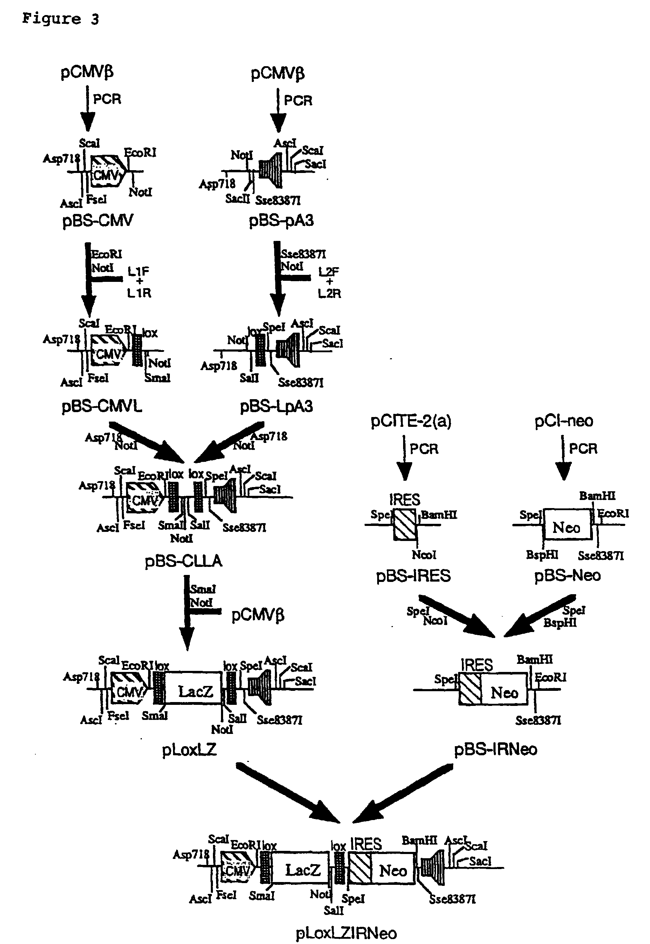

### 3-1) Cloning of CMV enhancer-promoter (Fig. 3)

The present inventors used the synthetic oligonucleotide CMF 
(TCAGGTACCGGCGCGCCAGTACTGGCCGGCCCGTTACATAACTTACGGTAAATGG; SEQ ID
NO: 15)
 containing the recognition sequences of restriction enzymes Asp718, AscI, ScaI, and FseI, and synthetic oligonucleotide CMR (TCCGAATTCGTACAATTCCGCAGCTTTTAGAGC; SEQ ID NO: 16) containing the recognition sequence of restriction enzyme EcoRI as the primers and the plasmid pCMVbeta (Clontech, USA) as the template to perform PCR to amplify and isolate the enhancer-promoter-intron region of pCMVbeta (enhancer and promoter of the immediate-early promoter of cytomegalovirus + intron of the SV40 virus, 0.8 kb). The fragments were cleaved with restriction enzymes Asp718 and EcoRI, then cloned between the Asp718-EcoRI sites of the pBluescriptII SK(-) plasmid (Stratagene). Nucleotide sequences of inserts were determined, and a clone bearing no mutation was selected. This plasmid was designated as "pBS-CMV."

### 3-2) Cloning of transcription termination region of SV40 virus late gene (Fig. 3)

The present inventors used the synthetic oligonucleotide PA3F (TTGCCGCGGCCTGCAGGCAGACATGATAAGATACATTGATG; SEQ ID NO: 17) containing the recognition sequences of restriction enzymes SacII and Sse8387I, and synthetic oligonucleotide PA3R (TCCGAGCTCAGTACTGGCGCGCCAAAAAACCTCCCACACCTCCCCCT, SEQ ID NO: 18) containing the recognition sequences of restriction enzymes SacI, ScaI, and AscI as the primers and the plasmid pCMVbeta as the template to perform PCR to amplify and isolate the transcription termination region (0.2 kb) of the SV40 virus late gene in pCMVbeta. After the fragments were cleaved with restriction enzymes SacI and SacII, they were cloned between the SacI-SacII sites of pBluescriptII SK(-). Nucleotide sequences of inserts were determined, and a clone bearing no mutation was selected. This plasmid was designated as "pBS-pA3."

### 3-3) Synthesis and cloning of loxP sequence (Fig. 3)

The synthetic oligonucleotides L1F (AATTCATAACTTCGTATAGCATACATTATACGAAGTTATCCCGGGGC; SEQ ID NO: 19) and L1R (GGCCGCCCCGGGATAACTTCGTATAATGTATGCTATACGAAGTTATG; SEQ ID NO: 20) containing the LoxP sequence and recognition sequences of restriction enzymes EcoRI, SmaI, and NotI were treated with T4 polynucleotide kinase to phosphorylate the 5'-termini. Both oligonucleotides were annealed and cloned between the EcoRI-NotI sites of pBS-CMV. Nucleotide sequences of inserts were determined, and a clone bearing no sequence error was selected. This plasmid was designated as "pBS-CMVL."

Synthetic oligonucleotides L2F 
(GGCCGCGTCGACATAACTTCGTATAGCATACATTATACGAAGTTATACTAGTCCTGCA; SEQ
ID NO: 21)
 and L2R (GGACTAGTATAACTTCGTATAATGTATGCTATACGAAGTTATGTCGACGC; SEQ ID NO: 22) containing the LoxP sequence and recognition sequences of restriction enzymes NotI, SalI, SpeI, and Sse8387I were similarly treated with T4 polynucleotide kinase, annealed, then cloned between the Sse8387I-NotI sites of pBS-PA3. Nucleotide sequences of inserts were determined, and a clone bearing no sequence error was selected. This plasmid was designated as "pBS-LpA3."

### 3-4) Preparation of plasmid vector for reporter construction (Fig. 3)

pBS-CMVL was cleaved with restriction enzymes Asp718 and NotI to isolate a 0.9 kb fragment containing the CMV promoter region and loxP sequence. pBS-LpA3 was cleaved with Asp718 and NotI to isolate a 3.2 kb fragment containing the loxP sequence, transcription termination region of SV40 virus late gene, and replication origin and selection marker of the plasmid. A plasmid in which both of the above fragments were ligated was isolated and designated as ^{"}pBS-CLLA." This plasmid has a unit containing the CMV enhancer-promoter-SV40 intron region-two loxPs-transcription termination region of SV40 virus late gene. Recognition sequences of restriction enzymes SmaI (or XmaI), NotI, and SalI are located between the two loxPs.

### 3-5) Preparation of reporter vector containing the β-galactosidase gene (Fig. 3)

After the plasmid pCMVbeta was cleaved with restriction enzymes SmaI, NotI, and PstI, a 3.4 kb fragment containing the β-galactosidase gene of *Escherichia coli* was isolated. This fragment was cloned between the SmaI-NotI sites of pBS-CLLA. This plasmid was designated as "pLoxLZ."

### 3-6) Preparation of plasmid expressing the β-galactosidase gene and G418 resistance gene (Fig. 3)

A plasmid simultaneously expressing the β-galactosidase (LacZ) gene and G418 resistance (Neo) gene of *E. coli* was prepared.

The G418 resistance gene ligated to the Internal Ribosome Entry Site (IRES) sequence was inserted between the second (3'-side) loxP sequence and transcription termination region of pLoxLZ. The IRES sequence having the ribosome binding sequence is often found in viruses. Genes located in its downstream can be efficiently translated once transcribed even if no CAP structure is nearby. Thus, IRES allows us to express plural gene products from a single transcription unit.

The present inventors used the synthetic oligonucleotide NeF (TCCACTAGTTCATGATTGAACAAGATGGATTGCAC; SEQ ID NO: 23) containing the recognition sequences of restriction enzymes SpeI and BspHI, and synthetic oligonucleotide NeR (TCCCGAATTCCCTGCAGGGGATCCTCAGAAGAACTCGTCAAGAAGGCG; SEQ ID NO: 24) containing the recognition sequences of restriction enzymes EcoRI, Sse8387I, and BamHI as the primers and the plasmid pCI-neo (Promega, USA) as the template to perform PCR to amplify and isolate the G418 resistance gene (0.8 kb) in pCI-neo. After the fragments were cleaved with restriction enzymes SpeI and EcoRI, they were cloned between the SpeI-EcoRI sites of pBluescriptII SK(-). Nucleotide sequences of inserts were determined, and a clone bearing no mutation was selected. This plasmid was designated as "pBS-Neo."

Using the synthetic oligonucleotide IRF (TCCACTAGTGTTATTTTCCACCATATTGCCGTC; SEQ ID NO: 25) containing the recognition sequence of restriction enzyme SpeI, and synthetic oligonucleotide IRR (TCAGGATCCCCATGGCCATGGTATTATCATCGT; SEQ ID NO: 26) containing the recognition sequences of restriction enzymes BamHI and NcoI as the primers and the plasmid pCITE-2(a) (Novagen, USA) as the template, PCR was performed to amplify and isolate the IRES region (0.5 kb) in pCITE-2(a). The fragments were cleaved with restriction enzymes SpeI and BamHI then cloned between the SpeI-BamHI sites of pBluescriptII SK(-). Nucleotide sequences of inserts were determined, and a clone bearing no mutation was selected. This plasmid was designated as "pBS-IRES."

pBS-IRES was cleaved with restriction enzymes SpeI and NcoI to isolate a 0.5 kb fragment containing the IRES region. This fragment was cloned between the SpeI-BspHI sites (located at the 5'-terminus of the G418 resistance gene) of pBS-Neo to prepare a plasmid in which the G418 resistance gene was linked to the 3'-terminus of the IRES region. This plasmid was designated as "pBS-IRNeo."

pBS-IRNeo was then cleaved with restriction enzymes SpeI and Sse8387I to isolate a 0.8 kb fragment containing the IRES region and G418 resistance gene. This fragment was then cloned between the SpeI-Sse8387I sites (located between the second loxP and transcription termination region of the SV40 virus late gene) of pLoxLZ. The plasmid thus obtained was designated as "pLoxLZIRNeo." This plasmid's structure is composed of the CMV enhancer-promoter-SV40 intron region-loxP-LacZ-loxP-IRES-Neo-SV40 virus late gene transcription termination region.

### 3-7) Preparation of reporter vector containing luciferase gene

The plasmid pGEM-luc (Promega, USA) was cleaved with restriction enzymes NotI and SalI to isolate a 1.7 kb fragment containing the firefly luciferase gene. This fragment was cloned between the NotI-SalI sites of pBS-CLLA. This plasmid was designated as "pLoxluc."

### 3-8) Preparation of vector containing herpes simplex virus-thymidine kinase gene

The present inventors used the synthetic oligonucleotide TKF (TGAGCCCGGGCCGCCACCATGGCTTCGTACCCCTGCCAT; SEQ ID NO: 27) containing the recognition sequence of restriction enzyme SrfI and synthetic oligonucleotide TKR2 (TCCTACTAGTGCGGCCGCTCAGTTAGCCTCCCCCATCTC; SEQ ID NO: 28) containing the recognition sequence of restriction enzyme NotI as the primers, and the plasmid pHSV106 (Lifetech, USA) as the template to perform PCR to amplify and isolate the translation sequence (HSVTK, 1.1 kb) of the thymidine kinase gene of herpes simplex virus type I in pHSV106. The fragments were cleaved with restriction enzymes SrfI and NotI then cloned between the SmaI-NotI sites of pBS-CLLA. Nucleotide sequences of inserts were determined, and a clone bearing no mutation was selected. This plasmid was designated as "pLoxTK."

### 3-9) Construction of adenovirus vector for reporter

pLoxLZ or pLoxluc was cleaved with the restriction enzyme ScaI to isolate a 4.6 kb or 2.7 kb DNA fragment containing the CMV enhancer-promoter-SV40 intron region-loxP-LacZ (or luciferase gene)-loxP-IRES-Neo-transcription termination region of the SV40 virus late gene. These fragments were inserted into the SwaI site of pAdex1cw by the method described in 1-7) to obtain recombinant cosmids. These cosmids were designated as "pAxYCMLLZ" or "pAxYCMLluc." Using these cosmids, recombinant adenoviruses containing the above-described units were prepared by the method described in 1-7). These recombinant adenoviruses were designated as "AxYCMLLZ" or "AxYCMLluc."

pLoxLZ and pLoxTK were cleaved with restriction enzymes EcoRI and SpeI to isolate 3.6 kb and 1.2 kb DNA fragments containing loxP-LacZ-loxP and loxP-HSVTK-loxP, respectively. Their terminal ends were blunted with a DNA blunting kit (Takarashuzo, Japan). These fragments were inserted into the SwaI site of pAdex1CA by the method described in 1-7) to obtain recombinant cosmids. Recombinant cosmids in which the direction of the CAG promoter coincided with that of the HSVTK gene were designated as "pAxCAYLLZ" and "pAxCAYLTK." Using these cosmids, recombinant adenoviruses containing the above-described units were prepared by the method described in 1-7). These recombinant adenoviruses were designated as "AxCAYLLZ" and "AxCAYLTK."

### Example 2 Preparation of cells for assessment

The present inventors attempted to insert the unit containing the CMV enhancer-promoter-SV40 intron region-loxP-LacZ-loxP-IRES-Neo-transcription termination region of the SV40 virus late gene into p53-deficient Saos2 cells derived from the human osteosarcoma (ATCC Cell Line and Hybridomas, 8th ed., 1994, p282, American Type Culture Collection).

The plasmid pLoxLZIRNeo was made linear by cleaving it with the restriction enzyme BsaI (which cleaves regions other than the above-described unit). Saos2 cells suspended in a K-PBS buffer (1 x 10⁷ cells/ml, 0.4 ml) (Iwamoto et al., Biomanual Series 4 - Idenshi Dounyu to Hatsugen-Kaisekihou (Methods of Gene Transfer, Expression and Analysis), 1994, 23-28, Yohdosha) and BsaI-cleaved "pLoxLZIRNeo" (0.01 mg) were placed in a 0.4-cm wide gene pulser cuvette (BioRad, USA). The mixture was then pulsed at 250 V and 0.95 mFD using a Gene Pulser II (BioRad). A medium (McCoy's 5A medium + 15% fetal bovine serum, 9 ml) was added to the suspension, and the mixture was cultured at 37°C Three days later, cells were trypsinized then suspended in a medium containing 0.8 mg/ml G418, placed in ten 10-cm dishes, and continuously cultured. Seventeen days later, several colonies were isolated, suspended in a medium (0.4 ml) containing 0.4 mg/ml G418, placed in a 48-well plate, and continuously cultured. Some of these G418-resistant clones were stained with X-gal to select clones having LacZ well expressed. Genomic DNA, which was cleaved with appropriate restriction enzymes, was prepared from some of these G418-resistant and LacZ (+) clones and blotted onto nylon membranes. These blotted membranes were hybridized to the labeled LacZ gene DNA, and the number of inserted units per cell was estimated from the label intensity. The clone judged to contain one inserted unit per cell (designated as A8a) was used in the succeeding experiments.

### Example 3 Expression test

### 1) Expression by the p53-dependent promoter

The induction capability of the p53-dependent promoter carried on the adenovirus vector was examined. Saos2 cells or Hs68 cells derived from the human fetal prepuce (ATCC Cell Line and Hybridomas, 8th ed., 1994, p282, American Type Culture Collection) that had been placed at 4 x 10⁵ cells per well into six-well plates on the previous day were infected with AxYp53RELZ and AxCAYp53 at the indicated multiplicity of infection (MOI). Infection was carried out according to the method of Kanegae et al. (Biomanual Series 4 - Idenshi Dounyu to Hatsugen-Kaisekihou (Methods of Gene Transfer, Expression and Analysis), 1994, p43-58, Yohdosha). Three days later, crude extracts of infected cells were prepared and assayed for the β-galactosidase activity using a Galactonplus kit (Tropics, USA). The results are shown in Table 1.

**Table 1**

| No. | Cell | AxCAYp53 (MOI) | AxYp53RELZ (MOI) | β-galactosidase (RLU/s/µg) |
|---|---|---|---|---|
| 1 | Saos2 (p53-) | 0 | 0 | 0 |
| 2 | | 0 | 100 | 12 |
| 3 | | 10 | 0 | 0 |
| 4 | | 10 | 100 | 284611 |
| 5 | Hs68 (p53+) | 0 | 0 | 0 |
| 6 | | 0 | 100 | 347741 |
| 7 | | 10 | 0 | 0 |
| 8 | | 10 | 100 | 452385 |

This promoter was strongly induced by p53, and in the case of p53-positive cells, the p53-dependent promoter was sufficiently induced by the endogenous p53 alone.

### 2) p53-dependent suppression of expression, assessment system 1

The suppression of p53-dependent LacZ expression was examined using p53-deficient cells A8a having in the genome the unit containing the CMV promoter, the loxP-Lacz gene, and the loxP-transcription termination signal as well as AxCAYp53 and AxYp53RELZ. The A8a cells that had been placed into six-well plates (4 x 10⁵ cells per well) on the previous day were infected with various recombinant adenoviruses at indicated MOIs. Seven days later, crude extracts of infected cells were prepared and assayed for β-galactosidase activity similarly as described in 1). The results expressed as the relative activity are shown in Table 2.

**Table 2**

| No. | Cell | AxCAYp53 (MOI) | AxYp53RELZ (MOI) | β-galactosidase (ratio) |
|---|---|---|---|---|
| 1 | A8a | 0 | 0 | 1 |
| 2 | | 0 | 100 | 0.91 |
| 3 | A8a | 30 | 0 | 1 |
| 4 | | 30 | 100 | 0.17 |

Expression suppression by AxYp53RELZ was not observed in p53-cells, but, when p53 was expressed, expression suppression by AxYp53RELZ was observed.

### 3) p53-Dependent suppression of expression, assessment system 2

p53-Dependent suppression of LacZ expression was examined using a recombinant adenovirus (AxYCMLLZ or AxYCMLluc) having the unit containing the CMV promoter-loxP-LacZ gene (or the luciferase gene)-loxP-transcription termination signal as well as AxCAYp53 and AxYp53RECre. Saos2 cells that had been placed into six-well plates (4 x 10⁵ cells per well) on the previous day were infected with AxCAYp53 and AxYp53RECre at the indicated MOIs. The next day, they were infected with AxYCMLLZ or AxYCMLluc. Five days later, crude extracts of infected cells were prepared and assayed for β-galactosidase activity similarly as described in 1), or assayed for luciferase activity by the assay system of Promega. The results expressed as the relative activity are shown in Tables 3 and 4.

**Table 3**

| No. | Cell | AxYCMLLZ (MOI) | AxCAYp53 (MOI) | AxYp53RECre (MOI) | β-galactosidase (ratio) |
|---|---|---|---|---|---|
| 1 | Saos2 | 10 | 0 | 0 | 1 |
| 2 | | 10 | 0 | 100 | 1.47 |
| 3 | Saos2 | 10 | 10 | 0 | 1 |
| 4 | | 10 | 10 | 100 | 0.09 |

**Table 4**

| No. | Cell | AxYCMLluc (MOI) | AxCAYp53 (MOI) | AxYp53RECre (MOI) | luciferase (ratio) |
|---|---|---|---|---|---|
| 1 | Saos2 | 3 | 0 | 0 | 1 |
| 2 | | 3 | 0 | 100 | 0.94 |
| 3 | Saos2 | 3 | 10 | 0 | 1 |
| 4 | | 3 | 10 | 100 | 0.12 |

Similarly as in 2), the expression suppression by AxYp53RECre was not observed in p53- cells, but, when p53 was expressed, significant expression suppression by AxYp53RECre was observed.

### 4) p53-Dependent suppression of expression, assessment system 3

The suppression of LacZ expression by p53 was examined using a recombinant adenovirus having the unit containing the CMV promoter-loxP-LacZ gene-loxP-transcription termination signal and various p53+ cells (Hs68 and IMR90 (derived from the human lung, ATCC Cell Line and Hybridomas, 8th ed., 1994, p100, American Type Culture Collection)) or p53- cells (Saos2 and J82 (derived from the human bladder cancer, ATCC Cell Line and Hybridomas, 8th ed., 1994, p239, American Type Culture Collection)). Various p53+ or p53- cells that had been placed into six-well plates (4 x 10⁵ cells per well) on the previous day were infected with AxYp53RECre at the indicated MOIs. On the following day, they were infected with AxYCMLLZ. Five days later, crude extracts of infected cells were prepared and assayed for β-galactosidase activity similarly as described in 3-1). The results expressed as the relative activity are shown in Table 5.

**Table 5**

| No. | Cell | AxYCMLLZ (MOI) | AxYp53RECre (MOI) | β-galactosidase (ratio) |
|---|---|---|---|---|
| 1 | Saos2 (p53-) | 3 | 0 | 1 |
| 2 | | 3 | 100 | 1.31 |
| 3 | J82 (p53-) | 3 | 0 | 1 |
| 4 | | 3 | 100 | 0.59 |
| 5 | Hs68 (p53+) | 3 | 0 | 1 |
| 6 | | 3 | 100 | 0.091 |
| 7 | IMR90 (p53+) | 3 | 0 | 1 |
| 8 | | 3 | 100 | 0.006 |

Almost no suppression of expression by AxYp53RECre was seen in p53-deficient cells Saos2 and J82, but significant suppression of expression by AxYp53RECre was observed in p53-expressing cells Hs68 and IMR90.

### 5) Potentiation of p53-dependent suppression of expression by antitumor agents

U2OS cells (p53+) that had been placed into six-well plates (6 x 10⁵ cells per well) on the previous day were infected with AxYp53RECre at the indicated MOIs. Various compounds (actinomycin D, doxorubicin hydrochloride, etoposide, and nocodazole, all from Sigma, USA) were then added at the indicated concentrations. On the next day, the compounds were removed, and AxCAYLLZ was infected at the indicated MOIs. Six days later, crude extracts of infected cells were prepared and assayed for β-galactosidase activity similarly as described in 3-1). Table 6 shows the effects of various compounds as the value obtained by dividing the enzyme activity when infected with AxYp53RECre by that under the non-infected conditions (described as remaining activity).

**Table 6**

| No. | Cell | AxCAYLLS (MOI) | pAxYp53RECre (MOI) | Compound added | Remaining β-galactosidase activity (%) |
|---|---|---|---|---|---|
| 1 | U2OS | 10 | 0 | None | 100 |
| 2 | | 10 | 30 | None | 17 |
| 3 | | 10 | 0 | 10 ng/ml actinomycin D | 100 |
| 4 | | 10 | 30 | 10 ng/ml actinomycin D | 2.4 |
| 5 | | 10 | 0 | 100 ng/ml doxorubicin | 100 |
| 6 | | 10 | 30 | 100 ng/ml doxorubicin | 0.5 |
| 7 | | 10 | 0 | 1000 ng/ml etoposide | 100 |
| 8 | | 10 | 30 | 1000 ng/ml etoposide | 0.6 |
| 9 | U2OS | 10 | 0 | None | 100 |
| 10 | | 10 | 10 | None | 68 |
| 11 | | 10 | 0 | 50 ng/ml nocodazole | 100 |
| 12 | | 10 | 10 | 50 ng/ml nocodazole | 31 |

Suppression effects of AxYp53RECre on the expression were enhanced in p53-normal cells by various antitumor agents.

### 6) Cell death specific to p53-deficient cells

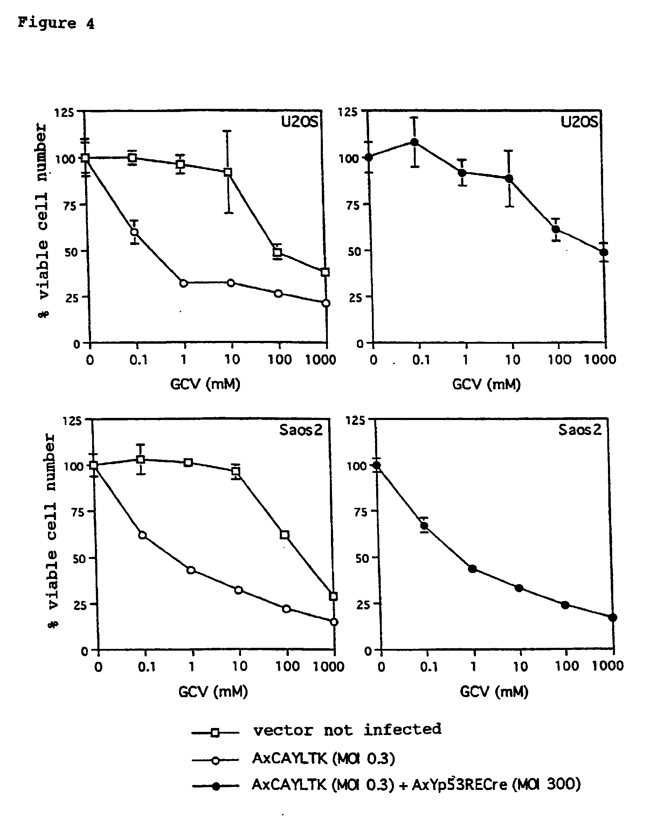

p53-Deficient Saos2 cells and p53-expressing U20S cells were inoculated then cultured in the presence of 50 ng/ml nocodazole (Sigma, USA) on the next day. Nocodazole was removed on the following day, and the cells were infected with AxYp53RECre at the indicated MOIs. On the day after that, the cells were further infected with AxCAYLTK at the indicated MOIs. Three days later, infected cells were placed into 96-well plates at a quantity of 2000 cells/well, and various concentrations of ganciclovir (Nippon Roche) were added thereto. Seven days later, the number of viable cells was counted using a Cell Titer 96 AQueous Non-Radioactive Cell Proliferation Assay (Promega, USA). Figure 4 shows the effects of the AxYp53RECre infection on the ganciclovir sensitivity of the cell. In this figure, "% viable cell number" refers to the relative number of cells compared to the number of cells in the absence of ganciclovir taken as 100. Mean values of five samples are shown. Error bars show standard deviations.

In p53-expressing U20S cells, the ganciclovir sensitivity of the cell was reduced by the AxYp53RECre infection, but in the p53-deficient Saos2 cells, no such effects were observed, proving that a combination of AxYp53RECre, AxCAYLTK, and ganciclovir caused cell death specific to p53-deficient cells.

### Industrial Applicability

The present invention provides a vector containing (1) a promoter functioning in the presence of a specific transcription factor and a gene encoding a recombinase present under the control of the promoter, and (2) an desired expressible gene and two target sequences of the recombinase located near the gene. In this case, the desired gene and the two target sequences are located in such a way that when recombination occurs between the two target sequences, the desired gene is not expressed.) This vector enables expressing an desired gene that is specific to cells free from a specific transcription factor.

Vectors according to this invention are preferable for various treatments, especially for cancer treatment. Specifically, 1) since vectors according to this invention control the gene expression in steps prior to transcription using a combination of a recombinase and its recognition sequences, they pose no problems in expression intensity and expression specificity such as caused in the system of Balmain et al. (Balmain et al., WO97/12970), and 2) since the vectors employ a system of irreversibly destroying gene expression, a switch OFF system, whereby a desired gene becomes physically non-expressible due to the expression of a catalytic amount of recombinase, they pose few problems with regard to the specificity and toxicity to normal cells that will be affected by a promoter to be used as in the above-described switch ON system of Sato et al. (Sato et al., 1997, the 3rd Annual Meeting of the JAPAN Society of Gene Therapy, Abstract p49). Therefore, use of the vectors of this invention will enable specific and safe therapy for a wide range of cancers.

## Claims

1. A vector comprising (1) a promoter functioning in the presence of a specific transcription factor and a gene encoding a recombinase present under the control of the promoter, and (2) an expressible desired gene and two target sequences of the recombinase present near the gene, wherein the desired gene and the two target sequences are so located that the desired gene will not be expressed when recombination occurs between the two target sequences.

2. The vector according to claim 1, wherein (1) a promoter functioning in the presence of a specific transcription factor and a gene encoding a recombinase present under the control of the promoter, and (2) an expressible desired gene and two target sequences of the recombinase present near the gene are present in the same molecule.

3. The vector according to claim 1, wherein (1) a promoter functioning in the presence of a specific transcription factor and a gene encoding a recombinase present under the control of the promoter, and (2) an expressible desired gene and two target sequences of the recombinase present near the gene are present in the different molecules.

4. The vector according to claim 1, wherein the desired gene is interposed between two target sequences of the recombinase.

5. The vector according to claim 4, wherein a promoter controlling the desired gene is interposed between two target sequences of the recombinase.

6. The vector according to claim 1, wherein the specific transcription factor is a translation product of a tumor suppressor gene.

7. The vector according to claim 6, wherein the tumor suppressor gene is the p53 gene.

8. The vector according to claim 1, wherein the recombinase is Cre, and the target sequence of recombinase is loxP.

9. The vector according to claim 1, wherein the desired gene is a gene encoding a suicide enzyme.

10. The vector according to claim 1, wherein the specific transcription factor is a tumor suppressor gene product, and the desired gene is a gene encoding a suicide enzyme.

11. The vector according to claim 10, wherein the tumor suppressor gene is the p53 gene.

12. A host cell transformed with the vector according to any one of claims 1 to 11.

13. A method for selectively exterminating cells free from a specific transcription factor, the method comprising introducing the vector according to any one of claims 1 to 11 into host cells *in vitro*.
